(19) Europäisches Patentamt
European Patent Office
Office européen des brevets

(11) EP 2 671 456 B1

(12) **EUROPEAN PATENT SPECIFICATION**

(45) Date of publication and mention of the grant of the patent:
**27.02.2019 Bulletin 2019/09**

(21) Application number: **12741768.1**

(22) Date of filing: **31.01.2012**

(51) Int Cl.:
*A61K 39/395* (2006.01) *A61P 35/02* (2006.01)
*C12N 9/10* (2006.01) *C12P 19/18* (2006.01)
*C12N 15/09* (2006.01) *A23L 7/10* (2016.01)
*A23L 7/104* (2016.01) *A23L 7/109* (2016.01)
*A21D 8/04* (2006.01) *C07H 15/04* (2006.01)

(86) International application number:
**PCT/JP2012/052082**

(87) International publication number:
**WO 2012/105532 (09.08.2012 Gazette 2012/32)**

(54) **NOVEL USE OF MALTOTRIOSYL TRANSFERASE**

NEUE VERWENDUNG VON MALTOTRIOSYL-TRANSFERASE

NOUVELLE UTILISATION DE MALTOTRIOSYL TRANSFÉRASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2011 JP 2011023281**
**26.07.2011 JP 2011162973**

(43) Date of publication of application:
**11.12.2013 Bulletin 2013/50**

(73) Proprietor: **Amano Enzyme Inc.**
**Nagoya-shi**
**Aichi 460-8630 (JP)**

(72) Inventors:
- **OKADA Masamichi**
  **Kakamigahara-shi**
  **Gifu 509-0109 (JP)**
- **YAMAGUCHI Shotaro**
  **Kakamigahara-shi**
  **Gifu 509-0109 (JP)**

(74) Representative: **Steinecke, Peter**
**Müller Fottner Steinecke**
**Rechtsanwalts- und Patentanwaltspartnerschaft mbB**
**Römerstrasse 16 b**
**52428 Jülich (DE)**

(56) References cited:
**EP-A1- 0 200 095** **WO-A1-2011/001722**
**WO-A1-2011/001722** **JP-A- H07 236 496**
**JP-A- 2008 194 024** **JP-A- 2008 194 024**
**JP-A- 2009 142 233** **JP-A- 2009 268 400**
**JP-A- 2010 202 884** **US-A- 5 387 516**
**US-A1- 2005 031 734** **US-A1- 2010 256 345**

- **MOON Y H ET AL: "Synthesis, Structure Analyses, and Characterization of Novel Epigallocatechin Gallate (EGCG) Glycosides Using the Glucansucrase from Leuconostoc mesenteroides B-1299CB", JOURNAL OF AGRICULTURAL AND FOOD CHEMISTRY, AMERICAN CHEMICAL SOCIETY, US, vol. 54, no. 4, 22 February 2006 (2006-02-22), pages 1230-1237, XP002696285, ISSN: 0021-8561, DOI: 10.1021/JF052359I [retrieved on 2006-01-21]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 671 456 B1

SEQUENCE LISTING

<110> AMANO ENZYME INC. OKADA, Masamichi YAMAGUCHI, Shotaro

<120> NOVEL USE OF MALTOTRIOSYL TRANSFERASE

<130> AE11001P

<150> JP P2011-023281
<151> 2011-02-04

<150> JP P2011-162973
<151> 2011-07-26

<160> 3

<170> PatentIn version 3.5

<210> 1
<211> 2304
<212> DNA
<213> Geobacillus sp.

<400> 1

```
atgtggaagg ttccaaaatt cattaaacaa tcatatctcg tttttcttct tgctctattg      60
ctctattctt catttggttt ttcgttttcc aggactgagg cgactacatc tacaggagct     120
ttaggaccag ttaccccgaa agacacaata tatcagatcg tgacggaccg ttttttttgat    180
ggcgacccat caaataacaa gcctcctggt tttgatccta ccctgtttga tgatccggac     240
ggcaataacc aggggaacgg aaaagattta aagttgtatc aaggcggtga tttccaagga     300
atcatagata aaattcctta tttaaaaaat atggggataa ctgccgtttg gatttccgct     360
ccttatgaaa atagggacac tgtgatagaa gattatcaat cagatggaag tattaatcgt     420
tggaccagtt tccacggtta ccatgcaaga aattattttg caactaacaa acattttggt     480
actatgaaag attttattag actacgcgat gctttgcatc aaaatggaat taaactagtg     540
atagattttg tatccaatca ttcgagtcgt tggcaaaacc cgacattgaa ttttgcgcct     600
gaagatggta aattatatga acctgacaag gatgcgaatg gcaattacgt atttgatgct     660
aatggagagc ctgcggatta caacggtgat ggaaaagttg agaatctcct ggcggatcca     720
cataacgacg tgaatggttt tttccatggt ctgggtgacc ggggcaacga tacttctcgt     780
tttggctacc gttacaaaga tctaggttct ttggctgatt attctcagga aaatgcacta     840
gtggttgaac atttggagaa agcagctaaa ttttggaaat caaaagggat cgatggtttt     900
cgacatgatg ccactttgca tatgaatcct gcatttgtga agggatttaa agatgcaatt     960
gattcagatg caggtggccc ggttacccat tttggtgaat ttttcattgg aagaccggat    1020
cccaagtatg atgagtaccg gacatttcct gaacgaacag gagtcaacaa cttggatttt    1080
gaatatttcc gtgcggccac aaacgcattt gggaactttt ctgaaacgat gagttccttt    1140
```

```
ggtgatatga tgatcaagac aagtaatgat tacatttatg aaaatcaaac agttactttc      1200
ttggataatc atgatgtaac aagatttcgc tatattcaac caaacgataa accttatcat      1260
gcagctctcg ctgtcttgat gacatcacgt gggattccta acatttatta cggaacagag      1320
caatatctga tgccgtcaga ctcaagtgac attgcgggtc ggatgtttat gcagacttct      1380
actaacttcg atgaaaatac cactgcatat aaagtcattc aaaagctttc aaacttaaga      1440
aaaaataatg aagctattgc ctatggaacc acagaaattt tatacagcac aaatgatgta      1500
ctggtcttta aaagacagtt ctatgataaa caagtaattg tagcggtaaa ccgacaaccg      1560
gatcaaacgt ttaccattcc ggagttagat acgactcttc cagtaggaac ctatagtgat      1620
gtactgggtg gactgttata tgggagttca atgagcgtaa ataatgtcaa cggtcaaaac      1680
aaaatttcta gctttacctt gtctggagga gaggtcaatg tctggtcgta taacccatca      1740
ttggggactt taactccaag gattggcgac gttatttcca ccatgggacg tcccggtaat      1800
accgtttaca tttacggtac tggattagga ggaagcgtaa cagtcaaatt cggttctact      1860
gttgctactg tggtgtcaaa cagcgatcaa atgattgagg ctatagttcc aaacactaat      1920
cctggaattc aaaatattac agttacaaaa ggatctgtaa ccagtgatcc tttccgatat      1980
gaggtcctat ccggcgatca ggtgcaagta atttttcatg tgaatgccac aacgaattgg      2040
ggggaaaaca tttatgttgt cggaaacatt ccagagttgg gaagctggga tccgaaccaa      2100
tcgtctgagg cgatgttaaa tccgaactat ccagaatggt tcttgccagt gagtgtgccc      2160
aagggagcta cttttgaatt caagtttatc aaaaaagata acaatggaaa tgtcatttgg      2220
gaaagcagga gcaacagagt atttaccgca ccgaacagtt cgaccggtac tattgacacc      2280
cctttatatt tttgggataa ctaa                                             2304
```

<210> 2
<211> 767
<212> PRT
<213> Geobacillus sp.

<400> 2

```
Met Trp Lys Val Pro Lys Phe Ile Lys Gln Ser Tyr Leu Val Phe Leu
1               5                   10                  15

Leu Ala Leu Leu Leu Tyr Ser Ser Phe Gly Phe Ser Phe Ser Arg Thr
            20                  25                  30

Glu Ala Thr Thr Ser Thr Gly Ala Leu Gly Pro Val Thr Pro Lys Asp
        35                  40                  45

Thr Ile Tyr Gln Ile Val Thr Asp Arg Phe Phe Asp Gly Asp Pro Ser
    50                  55                  60
```

```
Asn Asn Lys Pro Pro Gly Phe Asp Pro Thr Leu Phe Asp Asp Pro Asp
65                  70                  75                  80

Gly Asn Asn Gln Gly Asn Gly Lys Asp Leu Lys Leu Tyr Gln Gly Gly
                85                  90                  95

Asp Phe Gln Gly Ile Ile Asp Lys Ile Pro Tyr Leu Lys Asn Met Gly
            100                 105                 110

Ile Thr Ala Val Trp Ile Ser Ala Pro Tyr Glu Asn Arg Asp Thr Val
        115                 120                 125

Ile Glu Asp Tyr Gln Ser Asp Gly Ser Ile Asn Arg Trp Thr Ser Phe
    130                 135                 140

His Gly Tyr His Ala Arg Asn Tyr Phe Ala Thr Asn Lys His Phe Gly
145                 150                 155                 160

Thr Met Lys Asp Phe Ile Arg Leu Arg Asp Ala Leu His Gln Asn Gly
                165                 170                 175

Ile Lys Leu Val Ile Asp Phe Val Ser Asn His Ser Ser Arg Trp Gln
            180                 185                 190

Asn Pro Thr Leu Asn Phe Ala Pro Glu Asp Gly Lys Leu Tyr Glu Pro
        195                 200                 205

Asp Lys Asp Ala Asn Gly Asn Tyr Val Phe Asp Ala Asn Gly Glu Pro
    210                 215                 220

Ala Asp Tyr Asn Gly Asp Gly Lys Val Glu Asn Leu Leu Ala Asp Pro
225                 230                 235                 240

His Asn Asp Val Asn Gly Phe Phe His Gly Leu Gly Asp Arg Gly Asn
                245                 250                 255

Asp Thr Ser Arg Phe Gly Tyr Arg Tyr Lys Asp Leu Gly Ser Leu Ala
            260                 265                 270

Asp Tyr Ser Gln Glu Asn Ala Leu Val Val Glu His Leu Glu Lys Ala
        275                 280                 285

Ala Lys Phe Trp Lys Ser Lys Gly Ile Asp Gly Phe Arg His Asp Ala
    290                 295                 300

Thr Leu His Met Asn Pro Ala Phe Val Lys Gly Phe Lys Asp Ala Ile
```

```
305                 310                 315                 320

Asp Ser Asp Ala Gly Gly Pro Val Thr His Phe Gly Glu Phe Phe Ile
                325                 330                 335

Gly Arg Pro Asp Pro Lys Tyr Asp Glu Tyr Arg Thr Phe Pro Glu Arg
            340                 345                 350

Thr Gly Val Asn Asn Leu Asp Phe Glu Tyr Phe Arg Ala Ala Thr Asn
        355                 360                 365

Ala Phe Gly Asn Phe Ser Glu Thr Met Ser Ser Phe Gly Asp Met Met
    370                 375                 380

Ile Lys Thr Ser Asn Asp Tyr Ile Tyr Glu Asn Gln Thr Val Thr Phe
385                 390                 395                 400

Leu Asp Asn His Asp Val Thr Arg Phe Arg Tyr Ile Gln Pro Asn Asp
                405                 410                 415

Lys Pro Tyr His Ala Ala Leu Ala Val Leu Met Thr Ser Arg Gly Ile
            420                 425                 430

Pro Asn Ile Tyr Tyr Gly Thr Glu Gln Tyr Leu Met Pro Ser Asp Ser
        435                 440                 445

Ser Asp Ile Ala Gly Arg Met Phe Met Gln Thr Ser Thr Asn Phe Asp
    450                 455                 460

Glu Asn Thr Thr Ala Tyr Lys Val Ile Gln Lys Leu Ser Asn Leu Arg
465                 470                 475                 480

Lys Asn Asn Glu Ala Ile Ala Tyr Gly Thr Thr Glu Ile Leu Tyr Ser
                485                 490                 495

Thr Asn Asp Val Leu Val Phe Lys Arg Gln Phe Tyr Asp Lys Gln Val
            500                 505                 510

Ile Val Ala Val Asn Arg Gln Pro Asp Gln Thr Phe Thr Ile Pro Glu
        515                 520                 525

Leu Asp Thr Thr Leu Pro Val Gly Thr Tyr Ser Asp Val Leu Gly Gly
    530                 535                 540

Leu Leu Tyr Gly Ser Ser Met Ser Val Asn Asn Val Asn Gly Gln Asn
545                 550                 555                 560
```

Lys Ile Ser Ser Phe Thr Leu Ser Gly Gly Glu Val Asn Val Trp Ser
565 570 575

Tyr Asn Pro Ser Leu Gly Thr Leu Thr Pro Arg Ile Gly Asp Val Ile
580 585 590

Ser Thr Met Gly Arg Pro Gly Asn Thr Val Tyr Ile Tyr Gly Thr Gly
595 600 605

Leu Gly Gly Ser Val Thr Val Lys Phe Gly Ser Thr Val Ala Thr Val
610 615 620

Val Ser Asn Ser Asp Gln Met Ile Glu Ala Ile Val Pro Asn Thr Asn
625 630 635 640

Pro Gly Ile Gln Asn Ile Thr Val Thr Lys Gly Ser Val Thr Ser Asp
645 650 655

Pro Phe Arg Tyr Glu Val Leu Ser Gly Asp Gln Val Gln Val Ile Phe
660 665 670

His Val Asn Ala Thr Thr Asn Trp Gly Glu Asn Ile Tyr Val Val Gly
675 680 685

Asn Ile Pro Glu Leu Gly Ser Trp Asp Pro Asn Gln Ser Ser Glu Ala
690 695 700

Met Leu Asn Pro Asn Tyr Pro Glu Trp Phe Leu Pro Val Ser Val Pro
705 710 715 720

Lys Gly Ala Thr Phe Glu Phe Lys Phe Ile Lys Lys Asp Asn Asn Gly
725 730 735

Asn Val Ile Trp Glu Ser Arg Ser Asn Arg Val Phe Thr Ala Pro Asn
740 745 750

Ser Ser Thr Gly Thr Ile Asp Thr Pro Leu Tyr Phe Trp Asp Asn
755 760 765

<210> 3
<211> 733
<212> PRT
<213> Geobacillus sp.

<400> 3

Thr Thr Ser Thr Gly Ala Leu Gly Pro Val Thr Pro Lys Asp Thr Ile
1 5 10 15

```
Tyr Gln Ile Val Thr Asp Arg Phe Phe Asp Gly Asp Pro Ser Asn Asn
            20                  25                  30

Lys Pro Pro Gly Phe Asp Pro Thr Leu Phe Asp Asp Pro Asp Gly Asn
        35                  40                  45

Asn Gln Gly Asn Gly Lys Asp Leu Lys Leu Tyr Gln Gly Gly Asp Phe
    50                  55                  60

Gln Gly Ile Ile Asp Lys Ile Pro Tyr Leu Lys Asn Met Gly Ile Thr
65                  70                  75                  80

Ala Val Trp Ile Ser Ala Pro Tyr Glu Asn Arg Asp Thr Val Ile Glu
                85                  90                  95

Asp Tyr Gln Ser Asp Gly Ser Ile Asn Arg Trp Thr Ser Phe His Gly
            100                 105                 110

Tyr His Ala Arg Asn Tyr Phe Ala Thr Asn Lys His Phe Gly Thr Met
        115                 120                 125

Lys Asp Phe Ile Arg Leu Arg Asp Ala Leu His Gln Asn Gly Ile Lys
    130                 135                 140

Leu Val Ile Asp Phe Val Ser Asn His Ser Ser Arg Trp Gln Asn Pro
145                 150                 155                 160

Thr Leu Asn Phe Ala Pro Glu Asp Gly Lys Leu Tyr Glu Pro Asp Lys
                165                 170                 175

Asp Ala Asn Gly Asn Tyr Val Phe Asp Ala Asn Gly Glu Pro Ala Asp
            180                 185                 190

Tyr Asn Gly Asp Gly Lys Val Glu Asn Leu Leu Ala Asp Pro His Asn
        195                 200                 205

Asp Val Asn Gly Phe Phe His Gly Leu Gly Asp Arg Gly Asn Asp Thr
    210                 215                 220

Ser Arg Phe Gly Tyr Arg Tyr Lys Asp Leu Gly Ser Leu Ala Asp Tyr
225                 230                 235                 240

Ser Gln Glu Asn Ala Leu Val Val Glu His Leu Glu Lys Ala Ala Lys
                245                 250                 255

Phe Trp Lys Ser Lys Gly Ile Asp Gly Phe Arg His Asp Ala Thr Leu
            260                 265                 270
```

```
His Met Asn Pro Ala Phe Val Lys Gly Phe Lys Asp Ala Ile Asp Ser
        275                 280                 285

Asp Ala Gly Gly Pro Val Thr His Phe Gly Glu Phe Phe Ile Gly Arg
    290                 295                 300

Pro Asp Pro Lys Tyr Asp Glu Tyr Arg Thr Phe Pro Glu Arg Thr Gly
305                 310                 315                 320

Val Asn Asn Leu Asp Phe Glu Tyr Phe Arg Ala Ala Thr Asn Ala Phe
                325                 330                 335

Gly Asn Phe Ser Glu Thr Met Ser Ser Phe Gly Asp Met Met Ile Lys
            340                 345                 350

Thr Ser Asn Asp Tyr Ile Tyr Glu Asn Gln Thr Val Thr Phe Leu Asp
        355                 360                 365

Asn His Asp Val Thr Arg Phe Arg Tyr Ile Gln Pro Asn Asp Lys Pro
    370                 375                 380

Tyr His Ala Ala Leu Ala Val Leu Met Thr Ser Arg Gly Ile Pro Asn
385                 390                 395                 400

Ile Tyr Tyr Gly Thr Glu Gln Tyr Leu Met Pro Ser Asp Ser Ser Asp
                405                 410                 415

Ile Ala Gly Arg Met Phe Met Gln Thr Ser Thr Asn Phe Asp Glu Asn
            420                 425                 430

Thr Thr Ala Tyr Lys Val Ile Gln Lys Leu Ser Asn Leu Arg Lys Asn
        435                 440                 445

Asn Glu Ala Ile Ala Tyr Gly Thr Thr Glu Ile Leu Tyr Ser Thr Asn
    450                 455                 460

Asp Val Leu Val Phe Lys Arg Gln Phe Tyr Asp Lys Gln Val Ile Val
465                 470                 475                 480

Ala Val Asn Arg Gln Pro Asp Gln Thr Phe Thr Ile Pro Glu Leu Asp
                485                 490                 495

Thr Thr Leu Pro Val Gly Thr Tyr Ser Asp Val Leu Gly Gly Leu Leu
            500                 505                 510

Tyr Gly Ser Ser Met Ser Val Asn Asn Val Asn Gly Gln Asn Lys Ile
        515                 520                 525
```

```
Ser Ser Phe Thr Leu Ser Gly Gly Glu Val Asn Val Trp Ser Tyr Asn
    530                 535                 540

Pro Ser Leu Gly Thr Leu Thr Pro Arg Ile Gly Asp Val Ile Ser Thr
545                 550                 555                 560

Met Gly Arg Pro Gly Asn Thr Val Tyr Ile Tyr Gly Thr Gly Leu Gly
                565                 570                 575

Gly Ser Val Thr Val Lys Phe Gly Ser Thr Val Ala Thr Val Val Ser
            580                 585                 590

Asn Ser Asp Gln Met Ile Glu Ala Ile Val Pro Asn Thr Asn Pro Gly
        595                 600                 605

Ile Gln Asn Ile Thr Val Thr Lys Gly Ser Val Thr Ser Asp Pro Phe
    610                 615                 620

Arg Tyr Glu Val Leu Ser Gly Asp Gln Val Gln Val Ile Phe His Val
625                 630                 635                 640

Asn Ala Thr Thr Asn Trp Gly Glu Asn Ile Tyr Val Val Gly Asn Ile
                645                 650                 655

Pro Glu Leu Gly Ser Trp Asp Pro Asn Gln Ser Ser Glu Ala Met Leu
            660                 665                 670

Asn Pro Asn Tyr Pro Glu Trp Phe Leu Pro Val Ser Val Pro Lys Gly
        675                 680                 685

Ala Thr Phe Glu Phe Lys Phe Ile Lys Lys Asp Asn Asn Gly Asn Val
    690                 695                 700

Ile Trp Glu Ser Arg Ser Asn Arg Val Phe Thr Ala Pro Asn Ser Ser
705                 710                 715                 720

Thr Gly Thr Ile Asp Thr Pro Leu Tyr Phe Trp Asp Asn
                725                 730
```

## Description

### Technical Field

The present invention relates to a novel use of maltotriosyl transferase, and specifically to a method for producing an indigestible saccharide.

### Background Art

Known examples of industrially used glycosyltransferases include α-glucosidase (production of isomaltooligosaccharide or nigerooligosaccharide), β-fructofuranosidase (production of fructooligosaccharide or lactosucrose), β-galactosidase (production of galactooligosaccharide), α-glucosyltransferase (production of palatinose), cyclodextrin glucanotransferase (production of cyclodextrin or coupling sugar), and branching enzymes (production of highly branched cyclic dextrin). Alfa-glucosidase and branching enzymes act on polysaccharides and oligosaccharides containing α-1,4 bonds to catalyze transglucosylation. Alfa-glucosidase catalyzes transglucosylation of monosaccharides, and branching enzymes catalyze transglucosylation of oligosaccharides of four or more sugars or polysaccharides.

One of uses of enzymes is the production of processed food containing starch. Starch aging causes considerable problems such as the deterioration of storage stability. Starch aging is mostly caused by aging of amylose molecules contained in starch, more specifically association and insolubilization of the amylose molecules (Non-patent Document 1). Therefore, control of starch aging through depolymerization of starch was studied, which allowed control of starch aging to some degree. However, depolymerization impairs the original properties of starch. Furthermore, under this method, decomposed starch has increased reducing power, and is stained upon reaction with protein or amino acid under heating. Therefore, this method has limited uses (Patent Document 1). Accordingly, aging control without depolymerization of starch has been studied.

Under the above circumstances, the present applicant reported a novel glycosyltransferase which catalyzes the transglucosylation of maltotriose units (hereinafter referred to as "maltotriosyl transferase" or "the present enzyme") and a method for producing the same in a preceding patent application, and showed the examples of the uses. (Patent Document 2).

### Prior Art Documents

#### Patent Document

Patent Document 1: Japanese Unexamined Patent Application Publication No. 2001-294601
Patent Document 2: WO 2011/001722
Patent Document 3: Japanese Unexamined Patent Application Publication No. 54-49354
Patent Document 4: Japanese Unexamined Patent Application Publication No. 2008-194024
Patent Document 5: Japanese Unexamined Patent Application Publication No. 9-107900
Patent Document 6: WO 2008/136331
Patent Document 7: US 2005/031734 A1

#### Non-patent Document

Non-patent Document 1: Okada et Al., Denpun Kagaku (Journal of the Japanese Society of Starch Science), 30(2), p. 223-230 (1983)

### Summary of the Invention

#### Problems to be Solved by the Invention

The present invention is intended to provide a novel use of maltotriosyl transferase found by the inventors.

#### Means for Solving the Problem

In consideration of the above-described problems, the inventors carried out various investigations, and focused attention on the production and processing of rice cakes, and production of indigestible saccharide. The results of experiments indicated that the present enzyme has unexpectedly high heat resistance, sufficiently acts even when added before steaming the rice cake dough, and effectively prevents aging of rice cake. In addition, as a result of the study by the inventors, the present enzyme is useful for the production of indigestible saccharides, particularly because it allows the production on an industrial scale.

Rice cakes have a unique texture (chewy texture), but the texture is impaired as the starch ages with the lapse of time. Various measures are taken for preventing the deterioration of rice cake quality. Many of the measures use enzymes such as β amylase (for example, Patent Document 3) and transglucosidase (for example, Patent Document 4). When an enzyme is used, in consideration of action behavior and deactivation of the enzyme, the enzyme is usually added after moderately cooling the steamed rice cake dough (for example, Patent Document 5). However, such production method is complicated because it requires the step of cooling the rice cake dough after steaming (which requires temperature control, and may require the step of allowing the enzyme to react after adding the enzyme). The preceding patent application by the present applicant (Patent Document 2) describes the production process of rice cake using rice flour (Joshinko) as an example of the use of the present enzyme. In the process, the enzyme is added when the temperature of the dough becomes about 65°C, and thus the similar problem as above is posed.

Indigestible saccharides are also found to be useful, and various methods for producing them are developed. For example, Patent Document 4 describes the method for producing an indigestible saccharide using α-glucosyl transferase. In the production method, the temperature during reaction of the enzyme is low (40°C) likely due to the properties of the enzyme. The reaction at such a relatively low temperature involves high risks of microorganism contamination, and thus is not suitable to the production on industrial scale. On the other hand, the new production method established by the present study may be used under high temperature conditions because it uses an enzyme having high heat resistance, and thus reduces the risk of microorganism contamination. Accordingly, the method is also suitable to the production of indigestible saccharides on industrial scale. Patent Document 7 (US 2005/031734 A1) describes the production of indigestible saccharides based on a method comprising contacting ungelatinized starch comprising grain with a maltogenic starch liquefying enzyme such as alpha-amylase to produce maltose and subsequent contacting of said maltose with a transglucosidic enzyme, thereby, producing isomalto-oligosaccharides with increased frequency of 1,6-glycosidic links.

As a result of further study on indigestible saccharides, it was found that the present enzyme is effective for the increase of the indigestible saccharide content in beer or a beer-like beverage. It was also found that the present enzyme is markedly effective for the production of hetero-oligosaccharides and glycosides.

The present invention as characterized in the claims and described below is, as is evident from the above explanations, a solution to prior art, and brings about marked advantages.

[1] A method for producing an indigestible saccharide, including a step of allowing maltotriosyl transferase to act on a saccharide, wherein the saccharide is dextrin.

[2] The production method according to [1], wherein the step is carried out using one or more enzymes selected from the group consisting of cyclodextrin glucanotransferase, α-amylase (only those having transglycosylation activity), pullulanase, and isoamylase in combination with the maltotriosyl transferase.

[3] The production method according to [1] or [2], wherein the reaction temperature during the step is from 50°C to 70°C.

[4] The method for producing an indigestible saccharide according to [1] to [3], wherein the step is carried out in the presence of a receptor substrate, and a hetero-oligosaccharide is formed as the indigestible saccharide.

[5] The method for producing an indigestible saccharide according to any one of [4], wherein the receptor substrate is a monosaccharide and/or a sugar alcohol.

[6] The production method according to [4], wherein the receptor substrate is one or more sugars or sugar alcohols selected from the group consisting of D-xylose, D-fructose, D-glucose, L-fucose, L-sorbose, D-mannose, D-sorbitol, D-galactose, N-acetyl glucosamine, L-arabinose, D-ribose, and L-rhamnose.

[7] The method for producing an indigestible saccharide according to [1], wherein the saccharide is formed by saccharification of a saccharide ingredient used for producing beer or a beer-like beverage.

[8] The method for producing an indigestible saccharide according to [7], wherein the saccharide ingredient is one or more ingredients selected from the group consisting of malt, barley, wheat, rye, oat, corn, rice, kaoliang, potato, soybean, pea bean, chick-pea, and corn starch.

Brief Description of the Drawings

FIG. 1 is a graph showing the optimal temperature of the maltotriosyl transferase derived from *Geobacillus sp.* APC9669.

FIG. 2 is a graph showing the optimal pH of the maltotriosyl transferase derived from *Geobacillus sp.* APC9669.
FIG. 3 is a graph showing the thermostability of the maltotriosyl transferase derived from *Geobacillus sp.* APC9669.
FIG. 4 is a graph showing the pH stability of the maltotriosyl transferase derived from *Geobacillus sp.* APC9669.
FIG. 5 shows the result of SDS-PAGE of the maltotriosyl transferase. Lane 1: molecular weight marker, and lane 2: maltotriosyl transferase.

Description of Embodiments

(Term)

In the present invention, the term "DNA coding a protein" refers to the DNA which gives the protein upon expression, more specifically the DNA having a base sequence corresponding to the amino acid sequence of the protein. Accordingly, codon degeneracy is taken into consideration.

In the present description, the term "isolated" may be replaced with "purified". When the enzyme (maltotriosyl transferase) is derived from a natural material, the term "isolated" used for the enzyme means that the enzyme is substantially free of components of the natural material other than the enzyme (specifically substantially free of contaminant protein). Specifically, for example, in the isolated enzyme, the content of contaminant proteins is less than about 20%, preferably less than about 10%, more preferably less than about 5%, and even more preferably less than about 1% in the weight equivalence. On the other hand, when the enzyme is prepared by a genetic engineering technique, the term "isolated" means that the enzyme is substantially free of other components derived from the host cells or culture solution used. Specifically, for example, in the isolated enzyme, the content of contaminant components is less than about 20%, preferably less than about 10%, more preferably less than about 5%, and even more preferably less than about 1% in the weight equivalence. In the present description, the simple term "maltotriosyl transferase" means "isolated maltotriosyl transferase", unless it is evident that the term has a different meaning. The same applies to the term "the present enzyme" used in place of maltotriosyl transferase.

(Use of maltotriosyl transferase 1)

The enzyme maltotriosyl transferase (the present enzyme) has been disclosed by the inventors in the preceding patent application (see Patent Document 2 for more detail). As described in the preceding patent application, *Geobacillus sp.* APC9669 was found to produce maltotriosyl transferase, and the enzymatic properties were determined. The enzymatic properties thus determined are listed below.

(1) Action

The present enzyme is a maltotriosyl transferase, and acts on polysaccharides and oligosaccharides having $\alpha$-1,4 glucoside bonds as a binding mode to transfer maltotriose units to saccharides.

(2) Substrate specificity

The present enzyme favorably acts on soluble starch, amylose, amylopectin, maltotetraose, maltopentaose, and maltohexaose. On the other hand, the present enzyme does not act on $\alpha$-cyclodextrin, $\beta$-cyclodextrin, $\gamma$-cyclodextrin, maltotriose, and maltose.

(3) Molecular weight

The molecular weight of the present enzyme is about 83,000 (by SDS-PAGE).

(4) Optimum temperature

The optimum temperature for the present enzyme is about 50°C. The present enzyme exhibits high activity in the temperature range of about 45°C to 55°C. The optimum temperature was calculated by the below-described method for measuring maltotriosyl transferase activity (in 10 mmol/L MES buffer solution (pH 6.5)).

(5) Optimum pH

The optimum pH for the present enzyme is about 7.5. The present enzyme exhibits high activity in the pH range of about 6.5 to 8.0. The optimum pH is determined based on, for example, the measurement in a universal buffer solution.

(6) Thermostability

The present enzyme exhibits stable activity at 65°C or lower. The present enzyme keeps 90% or higher level of activity even after treatment for 30 minutes at 65°C in a 10 mmol/L MES buffer solution (pH 6.5).

(7) pH stability

The present enzyme exhibits stable activity in a wide pH range of 5.0 to 10.0. More specifically, the enzyme keeps 85% or higher level of activity after treatment for 30 minutes at 40°C, as long as the pH of the enzyme solution used for the treatment is within the above range.

(8) Isoelectric point

The isoelectric point of the present enzyme is about 4.5 (by Ampholine electrophoresis).

As shown in the prior patent application, when the maltotriosyl transferase produced by *Geobacillus sp.* APC9669 acts on maltotetraose as substrate, it gives a ratio between the maltoheptaose production rate and maltotriose production rate of 9:1 to 10:0 at any substrate concentration ranging from 0.67 to 70% (w/v), wherein maltoheptaose is a transglycosylation product and maltotriose is a decomposition product, respectively. In other words, the rate of transglucosylation is far higher over the wide substrate concentration range, and the maltoheptaose production rate was 90% or more, taking the sum of the maltoheptaose and maltotriose production rates as 100%. The rates were compared based on the molar ratios of the products.

The present enzyme is preferably a maltotriosyl transferase derived from *Geobacillus sp.* APC9669. The term "maltotriosyl transferase derived from *Geobacillus sp.* APC9669" in this case means a maltotriosyl transferase produced by *Geobacillus sp.* APC9669 (wild strain or mutant strain), or a maltotriosyl transferase obtained by a genetic engineering technique using the maltotriosyl transferase gene of *Geobacillus sp.* APC9669 (wild strain or mutant strain). Accordingly, "maltotriosyl transferase derived from *Geobacillus sp.* APC9669" includes the recombinants produced by host microorganisms into which the maltotriosyl transferase gene (or the modified version of the gene) obtained from *Geobacillus sp.* APC9669, *"Geobacillus sp.* APC9669 has been introduced.

For convenience of explanation, *Geobacillus sp.* APC9669 which is the source of the present enzyme is referred herein the bacterium producing the present enzyme. The APC9669 strain is deposited on the below-described depository, and is readily available therefrom.

Depository institution: Patent Microorganisms Depository, NITE Biotechnology Development Center (2-5-8, Kazusakamatari, Kisarazu-shi, Chiba, 292-0818, Japan)
Date of deposit (date of receipt): June 2, 2009
Accession number: NITE BP-770

After culturing the *Geobacillus sp.* APC9669, the present enzyme is collected from the culture solution and/or bacterial cells. The culture method may be liquid culture or solid culture, and preferably liquid culture. The conditions of liquid culture are described below.

The medium is not particularly limited as long as it is suitable for growing the microorganism used. Examples of the medium include those containing a carbon source such as glucose, sucrose, genthiobiose, soluble starch, glycerol, dextrin, molasses, or an organic acid, in addition, ammonium sulfate, ammonium carbonate, ammonium phosphate, ammonium acetate, or a nitrogen source such as peptone, yeast extract, corn steep liquor, casein hydrolysate, bran, or meat extract, in addition an inorganic salt such as a potassium salt, a magnesium salt, a sodium salt, a phosphate, a manganese salt, an iron salt, or a zinc salt. In order to accelerate the growth of the microorganism, a vitamin and an amino acid may be added to the medium. The pH of the medium is adjusted to, for example, about 3 to 10, and preferably about 7 to 8, and the incubation temperature is normally from about 10 to 80°C, preferably about 30 to 65°C. The microorganism is cultured for about 1 to 7 days, preferably for about 2 to 4 days under aerobic conditions. The culture method may be, for example, a shake culture method or an aerobic deep culture method using a jar fermenter.

After culturing under the above-described conditions, a maltotriosyl transferase is collected from the culture solution or bacterial cells. When the enzyme is collected from the culture solution, for example, the culture supernatant is subjected to, for example, filtration or centrifugation thereby removing insoluble matter, followed by separation and purification through an appropriate combination of, for example, concentration using an ultrafiltration membrane, salting out by ammonium sulfate precipitation, dialysis, and various chromatography procedures such as ion exchange chromatography, and thus obtaining the present enzyme.

On the other hand, when the enzyme is collected from bacterial cells, the bacterial cells are crushed by, for example, pressurization or ultrasonication, and then subjected to separation and purification in the same manner as described-above, and thus obtaining the present enzyme. Alternatively, the bacterial cells may be collected in advance from the culture solution by, for example, filtration or centrifugation, and then the above-described procedure (crushing of bacterial cells, separation, and purification) may be carried out.

Confirmation of expression and identification of the expression product are readily achieved using an antibody against the maltotriosyl transferase. Alternatively, the expression may be confirmed by measuring the maltotriosyl transferase activity.

The maltotriosyl transferase for the present invention according to one embodiment contains the amino acid sequence set forth in SEQ ID NO: 3. This amino acid sequence is the amino acid sequence set forth in SEQ ID NO: 2 excluding the signal peptide portion. The amino acid sequence set forth in SEQ ID NO: 2 was deduced from the base sequence (SEQ ID NO: 1) of the gene obtained by cloning *Geobacillus sp.* APC9669. In general, when the amino acid sequence of a certain protein is partially modified, the modified protein may have equivalent function to the unmodified protein. More specifically, modification of the amino acid sequence does not substantially influence the function of the protein, and thus the function of the protein may be maintained before and after the modification. Accordingly, for the present invention, use of a protein which is composed of an amino acid sequence equivalent to the amino acid sequence set forth in SEQ ID NO: 3, and has maltotriosyl transferase activity (hereinafter, also referred to as "equivalent protein") is also contemplated. The term "equivalent amino acid sequence" in this case means an amino acid sequence which is partially different from the amino acid sequence set forth in SEQ ID NO: 3, but the difference does not substantially influence the function of the protein (maltotriosyl transferase activity). The term "maltotriosyl transferase activity" means the activity for polysaccharides and oligosaccharides having $\alpha$-1,4 glucoside bonds as a binding mode to transfer the maltotriose units to saccharides. The degree of the activity is not particularly limited as long as the function of a maltotriosyl transferase can be exhibited, but is preferably equivalent to or higher than that of the protein composed of the amino acid sequence set forth in SEQ ID NO: 3.

The term "partial difference in the amino acid sequence" typically means mutation (change) in the amino acid sequence caused by deletion or substitution of one to several (up to, for example, 3, 5, 7, or 10) amino acids composing the amino acid sequence, or addition, insertion, or combination thereof of one to several (up to, for example, 3, 5, 7, or 10) amino acids. The difference in the amino acid sequence is acceptable as long as the maltotriosyl transferase activity is maintained (the activity may be varied to a degree). As long as the conditions are satisfied, the position of the difference in the amino acid sequence is not particularly limited, and the difference may arise in a plurality of positions. The term "plurality" means, for example, a number corresponding to less than about 30%, preferably less than about 20%, more preferably less than about 10%, even more preferably less than about 5% of the total amino acids, and most preferably less than about 1%. More specifically, the equivalent protein has, for example, about 70% or more, preferably about 80% or more, even more preferably about 90% or more, even more preferably about 95% or more, and most preferably about 99% or more identity with the amino acid sequence set forth in SEQ ID NO: 3.

Preferably, the equivalence protein is obtained by causing conservative amino acid substitution in an amino acid residue which is not essential for maltotriosyl transferase activity. The term "conservative amino acid substitution" means the substitution of an amino acid residue with another amino acid residue having a side chain with similar properties. Amino acid residues are classified into several families according to their side chains, such as basic side chains (for example, ricin, arginine, and histidine), acidic side chains (for example, aspartic acid and glutamic acid), uncharged polar side chains (for example, glycine, asparagine, glutamine, serine, threonine, tyrosine, and cysteine), nonpolar side chains (for example, alanine, valine, leucine, isoleucine, proline, phenylalanine, methionine, and tryptophan), $\beta$ branched side chains (for example, threonine, valine, and isoleucine), and aromatic side chains (for example, tyrosine, phenylalanine, tryptophan, and histidine). Conservative amino acid substitution is preferably the substitution between amino acid residues in one family.

The "equivalent protein" may have additional properties. For example, the equivalent protein may have higher stability than the protein composed of the amino acid sequence set forth in SEQ ID NO: 3, may perform a different function performed only at low temperatures and/or high temperatures, or may have a different optimum pH.

The identity (%) between the two amino acid sequences may be determined by, for example, the following procedure. Firstly, the two sequences are aligned for optimal comparison (for example, a gap may be introduced into the first sequence thereby optimizing the alignment with the second sequence). When the molecule at the specific position in the first sequence (amino acid residue) is the same as the molecule at the corresponding position in the second sequence, the molecules at the positions are regarded as identical. The sequence identity is the function of the number of the identical positions common to the two sequences (more specifically, identity (%) = number of identical positions / total number of positions $\times$ 100), and preferably the number and size of the gaps required for the optimization of the alignment are taken into consideration. Comparison of the two sequences and determination of the identity are achieved using a mathematic algorithm. Specific examples of the mathematic algorithm usable for the comparison of the sequences include, but not limited to, the algorithm described in Karlin and Altschul (1990) Proc. Natl. Acad. Sci. USA 87:2264-68, and modified in Karlin and Altschul (1993) Proc. Natl. Acad. Sci. USA 90:5873-77. These algorithms are incorporated into NBLAST Program and XBLAST Program (version 2.0) described in Altschul et al. (1990) J. Mol.

Biol. 215:403-10. For example, under XBLAST Program, when BLAST polypeptide is searched under conditions that score = 50 and wordlength = 3, an amino acid sequence with a high identity can be obtained. Gapped BLAST described in Altschul et al. (1997), Amino Acids Research 25(17): 3389-3402 can be used for obtaining a gap alignment for comparison. When BLAST and Gapped BLAST are used, default parameters of corresponding programs (for example, XBLAST and NBLAST) may be used. For more detailed information, see, for example, the website of NCBI. Other examples of the mathematic algorithm usable for the comparison of the sequences include the algorithm described in Myersand Miller (1988) Comput Appl BioSci. 4: 11-17. These algorithms are incorporated into, for example, ALIGN Program usable in GENESTREAM Network Server (IGH Montpellier, France) or ISREC Server. When ALIGN program is used for the comparison of amino acid sequences, for example, a PAM120 weight residue table is used, wherein the gap length penalty is 12, and the gap penalty is 4. The identity between the two amino acid sequences is determined according to GAP Program of GCG software package using Blossom 62 matrix or PAM250 matrix, wherein the gap weight is 12, 10, 8, 6, or 4, and the gap length weight is = 2, 3, or 4.

The present enzyme may be a portion of a larger protein (for example, a fused protein). Examples of the sequence added to a fused protein include the sequences useful for purification of multiple histidine residues, and addition sequences which ensures stability in recombination production.

The present enzyme having the above-described amino acid sequence is readily prepared by a genetic engineering technique. For example, an appropriate host cell (for example, Escherichia coli) is transformed by a DNA coding the present enzyme (specific example is a DNA having nucleotide sequence of SEQ ID NO: 1), and the protein expressed in the transformant is collected, and thereby preparing the present enzyme. The collected protein is treated as appropriate according to the intended use. The present enzyme thus obtained as a recombinant protein may be subjected to various modifications. For example, the present enzyme composed of a recombinant protein linked to any peptide or protein can be obtained by producing a recombinant protein using a vector into which a DNA coding the present enzyme has been inserted together with other appropriate DNA. In addition, modification for causing addition of a sugar chain and/or a lipid, or N- or C-terminal processing may be carried out. These modifications allow, for example, extraction of a recombinant protein, simplification of purification, or addition of biological functions.

(Use of maltotriosyl transferase 2: method for producing indigestible saccharide and uses therefor)

The present invention relates to providing a method for producing an indigestible saccharide as a use of maltotriosyl transferase as characterized in the claims, and also the uses of the indigestible saccharide produced by the production method. The method for producing an indigestible saccharide according to the present invention uses the same maltotriosyl transferase (the present enzyme) as the first aspect, preferably the maltotriosyl transferase disclosed by the present applicant in the preceding patent application (see Patent Document 2 for more details). In more detail, the method for producing an indigestible saccharide according to the present invention includes a step of allowing maltotriosyl transferase (preferably the present enzyme) to act on a saccharide as the substrate.

The "saccharide" to which the enzyme is acted on is a polysaccharide or oligosaccharide (for example, amylose, amylopectin, or any oligosaccharide) having $\alpha$-1,4 glucoside bonds. The "saccharide" in the present invention may be a mixture of two or more polysaccharides or oligosaccharides. According to the present invention "saccharide" is a dextrin (starch hydrolysate). Dextrins are usually obtained by the action of $\alpha$-amylase on starch, or acidolysis of starch. The starch to be the ingredient is not particularly limited. Examples of starch include corn starch, potato starch, wheat starch, rise starch, tapioca starch, and cassava starch. Two or more kinds of starch may be used in combination. At present, corn starch is particularly preferred from the viewpoints of supply and cost. The dextrin may be selected from various kinds of commercially available dextrin (starch hydrolysate).

In the present description, the term "indigestible saccharide" means a saccharide containing an indigestible component. The indigestible component content is not particularly limited. The production method according to the present invention can produce an indigestible saccharide having a relatively high indigestible component content (50% to 60% or more). In the present description, the term "indigestible saccharide" includes the terms "indigestible dextrin", "indigestible starch", and "indigestible oligosaccharide". For example, those produced using a dextrin as the substrate may be referred to as "indigestible dextrin", focusing on the substrate as an ingredient.

The loading of the present enzyme is not particularly limited, and may be from 1 U to 40 U for 1 g of saccharide.

The temperature during the enzyme reaction is not particularly limited, but preferably from 30°C to 70°C, thereby allowing the present enzyme to sufficiently act. On the other hand, the temperature is preferably higher for reducing the risk of microorganism contamination, so that the reaction temperature is, for example, from 50°C to 70°C.

As described in examples, the combination of the present enzyme and cyclodextrin glucanotransferase and/or pullulanase increased the formation of the indigestible component. These two enzymes used in combination do not form indigestible components by themselves, but the combined use with the present enzyme achieved a synergistic effect, and increased the indigestible component. On the basis of this finding, according to one embodiment of the present invention, the present enzyme is used in combination with one or more enzymes selected from the group consisting of cyclodextrin glucanotransferase, α-amylase (only those having transglycosylation activity), pullulanase, and isoamylase, and the combination is allowed to act on a dextrin. Alpha-amylase likely achieves the same effect as cyclodextrin glucanotransferase as long as it has transglycosylation activity, and thus may be used in combination with the present enzyme. Isoamylase shows a common action with pullulanase, and thus may be used in combination with the present enzyme. Examples of the cyclodextrin glucanotransferase include TORUZYME 3.0L (manufactured by NOVOZYMES), CONTIZYME (manufactured by Amano Enzyme Inc.), and those derived from *Geobacillus stearothermophilus.* Examples of the α-amylase having transglycosylation activity include the amylase derived from *Bacillus circulans* (Patent Document 4). Examples of the pullulanase include KLEISTASE PL45 (manufactured by Amano Enzyme Inc.), and Pullulanase "AMANO" 3 (manufactured by Amano Enzyme Inc.). Examples of the isoamylase include the isoamylase derived from *Pseudomonas sp.* (manufactured by Megazyme), and GODO-FIA (manufactured by GODO SHUSEI Co., Ltd.). The usage of the enzymes used in combination with the present enzyme may be established based on, for example, a preliminary experiment, with reference to the usage shown in the examples. For example, the usage of cyclodextrin glucanotransferase is from 0.1 mg to 20 mg for 1 g of the substrate, and the usage of pullulanase is from 0.1 mg to 10 mg for 1 g of the substrate.

The production method of the present invention may be used for the production of a hetero-oligosaccharide. When a hetero-oligosaccharide is produced, maltotriosyl transferase (preferably the present enzyme) is allowed to act on a saccharide in the presence of a receptor substrate. The saccharide is not particularly limited, and may be, for example, maltooligosaccharide, dextrin, or starch. Examples of the maltooligosaccharide include maltotetraose, maltopentaose, and maltohexaose. The receptor substrate is also not particularly limited, and may be, for example, monosaccharide or sugar alcohol. Examples of the monosaccharide and sugar alcohol include D-xylose, D-fructose, D-glucose, L-fucose, L-sorbose, D-mannose, D-sorbitol, D-galactose, N-acetyl glucosamine, L-arabinose, D-ribose, and L-rhamnose. The loading of the present enzyme is not particularly limited, and may be from 1 U to 400 U for 1 g of the substrate. The temperature during enzyme reaction is not particularly limited, and preferably from 30°C to 70°C, thereby allowing the present enzyme to sufficiently act.

According to one embodiment, the production method of the present invention is carried out as a part of the production process of beer or a beer-like beverage. In typical cases, the preparation step (saccharification step) of beer or a beer-like beverage is carried out after the addition of maltotriosyl transferase, or maltotriosyl transferase is added to the saccharified solution obtained after the preparation step, thereby carrying out the enzyme reaction. In this manner, the maltotriosyl transferase is allowed to act on the saccharide formed by saccharification of the saccharide ingredient used in beer or a beer-like beverage. The beer or a beer-like beverage in the present invention is not limited to the beer under the Liquor Tax Act, and include general brews such as low-malt beer and general beer-taste beverages which are brewed using a beer yeast. Low alcohol beer, non-alcohol beer, and the third beer are also included in "beer or beer-like beverages". Examples of the saccharide ingredient include malt, barley, wheat, rye, oat, corn, rice, kaoliang, potato, soybean, pea bean, chick-pea, and corn starch. As necessary, two or more ingredients are used in combination. In the production method according to this embodiment, beer or a beer-like beverage having a high indigestible saccharide (dietary fiber) content is obtained. In the production process of beer or a beer-like beverage, an enzyme such as glucoamylase, α-amylase, β-amylase, or pullulanase may be used in combination. These enzymes are used typically for the reduction of saccharides. These enzymes are added, for example, during the reaction of the maltotriosyl transferase.

The present application also provides a composition containing the indigestible saccharide produced by the production method of the present invention. The composition is not particularly limited as to its use, and is preferably used for medicine, quasi-drug, or food. More specifically, a pharmaceutical composition, a quasi-drug composition, and a food composition containing the indigestible saccharide produced by the production method of the present invention are provided.

The pharmaceutical composition and quasi-drug composition may be formulated according to a common procedure. The formulation may contain other pharmaceutically acceptable other components (for example, carrier, excipient, disintegrating agent, buffer, emulsifying agent, suspending agent, soothing agent, stabilizer, preservative, antiseptic, and normal saline solution). Examples of the excipient include lactose, starch, sorbitol, D-mannitol, and white sugar. Examples of the disintegrating agent include starch, carboxymethyl cellulose, and calcium carbonate. Examples of the buffer include phosphates, citrates, and acetates. Examples of the emulsifying agent include gum arabic, sodium alginate, and tragacanth. Examples of the suspending agent include glyceryl monostearate, aluminum monostearate, methyl cellulose, carboxymethyl cellulose, hydroxymethyl cellulose, and sodium lauryl sulfate. Examples of the soothing agent include benzyl alcohol, chlorobutanol, and sorbitol. Examples of the stabilizer include propylene glycol and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

The dosage form of the formulation is also not particularly limited. The pharmaceutical composition or quasi-drug composition may be provided in the form of, for example, pellets, powder, fine grains, granules, capsules, syrup, injection, external preparation, or suppository.

The pharmaceutical composition contains an active ingredient in an amount necessary to achieve the expected therapeutical or prophylactic benefit (more specifically therapeutically effective amount). In addition, the quasi-drug composition contains an active ingredient in an amount necessary to achieve the expected improving or prophylactic benefit. The amount of the active ingredient contained in the pharmaceutical composition or quasi-drug composition is, for example, about 0.1% by weight to about 95% by weight depending on the dosage form or shape, thereby achieving a desired dose.

Examples of the "food composition" include common food (grains, vegetable, meat, various processed food, confectionery, milk, refreshing beverage, alcohol beverage (for example, beer or beer-like beverage), food supplements (dietary supplements, nutrition drinks), food additives, pet food, and pet dietary supplements. Food supplements or food additives may be in the form of powder, granules, tablets, paste, or liquid. The form of a food composition makes it easy to routinely or continuously take the active ingredient (indigestible saccharide). The food composition preferably contains the active ingredient in an amount effective to achieve therapeutical or prophylactic benefit. The loading may be established in consideration of, for example, the disease state, health condition, age, sex, body weight of the subject.

[Example]

<Measurement method of maltotriosyl transferase activity>

The activity of the maltotriosyl transferase was measured as described below. More specifically, 0.5 mL of an enzyme solution was added to 2 mL of a 10 mmol/L MES buffer solution (pH 6.5) containing 1% maltotetraose (manufactured by Hayashibara Co., Ltd.), and allowed to stand at 40°C for 60 minutes. After standing, the mixture was heated for 5 minutes in a boiling water bath, and cooled in running water. The glucose thus formed was quantified using Glucose CII-Test Wako (manufactured by Wako Pure Chemical Industries, Ltd.). Under these conditions, the amount of the enzyme forming 1 μmol of glucose in 2.5 mL of the reaction solution for 1 minute was set at 1 unit.

<Confirmation method of activity of maltotriosyl transferase>

In addition to the above-described <Measurement method of maltotriosyl transferase activity>, the activity of maltotriosyl transferase was confirmed as described below. More specifically, 15 μL of 1.0 u/mL enzyme solution was added to 985 μL of 5 mmol/L acetic acid buffer solution (pH 6.0) containing 10.3 mmol/L maltotetraose (manufactured by Hayashibara Co., Ltd.), and allowed to stand at 50°C for 1, 2, and 3 hours. After standing, the mixture was heated in a boiling water bath for 5 minutes, and then cooled in running water. The cooled reaction solution was desalted as needed using cationic and anionic resins, and the reaction solution was analyzed by HPLC. In the analysis, the HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the consumption rate and production rate were calculated. For the purified maltotriosyl transferase, for example, the production rate ratio was heptaose : triose = about 92 : about 8.

As described in the preceding patent application (Patent Document 2), maltotriosyl transferase from *Geobacillus sp.* APC9669 was successfully obtained. The method for obtaining the enzyme and characteristics of the enzyme are cited from Patent Document 2 and described below.

1. Production and purification of maltotriosyl transferase from *Geobacillus sp.* APC9669

*Geobacillus sp.* APC9669 was cultured under shaking at 45°C for 2 days using a liquid culture medium having the components shown in Table 1. The culture supernatant thus obtained was concentrated five-fold using a UF membrane (AIP-1013D, manufactured by Asahi Kasei Corporation), and then ammonium sulfate was added to give a 50% saturated concentration. The precipitate fraction was dissolved again in 20 mmol/L tris-hydrochloride buffer solution (pH 8.0) containing 5 mmol/L calcium chloride, and subsequently ammonium sulfate was added to give a final concentration of 0.5 mol/L. The precipitate thus formed was removed by centrifugation, the remaining solution was passed through an HiLoad 26/10 Phenyl Sepharose HP column (manufactured by GE Healthcare) which had been equilibrated with 20 mmol/L tris-hydrochloride buffer solution (pH 8.0) containing 0.5 mol/L ammonium sulfate and 5 mmol/L calcium chloride, and the adsorbed maltotriosyl transferase protein was eluted by linear concentration gradient of ammonium sulfate from 0.5 mol/L to 0 mol/L.

[Table 1]

| Culture medium for producing maltotriosyl transferase | |
|---|---|
| | (w/v) |
| Yeast extract | 1.5% |
| Soybean peptone | 0.5% |
| Sodium chloride | 0.5% |
| Soluble starch | 0.4% |

The collected fraction containing active maltotriosyl transferase was concentrated using a UF membrane, and the buffer solution was replaced with a 20 mmol/L tris-hydrochloride buffer solution (pH 8.0) containing 5 mmol/L calcium chloride. The sample with a new buffer solution was passed through an HiLoad 26/10 Q Sepharose HP column (manufactured by GE Healthcare) which had been equilibrated with 20 mmol/L tris-hydrochloride buffer solution (pH 8.0) containing 5 mmol/L calcium chloride, and the adsorbed maltotriosyl transferase protein was eluted by linear concentration gradient of NaCl from 0 mol/L to 1 mol/L.

Furthermore, the collected fraction containing active maltotriosyl transferase was concentrated using a UF membrane, the buffer solution was replaced with a 50 mM phosphate buffer solution (pH 7.2) containing 0.15 M NaCl, and then the sample was passed through an HiLoad 26/60 Superdex 200 pg column (manufactured by GE Healthcare) which had been equilibrated with a 50 mM phosphate buffer solution (pH 7.2) containing 0.15 M NaCl, and eluted with the buffer. The fraction containing active maltotriosyl transferase was collected, desalted and concentrated using an ultrafiltration membrane, and thus a purified enzyme preparation was obtained. The purified enzyme thus obtained was subjected to the study of the following properties.

The results of the purification at each step are shown in Table 2. The specific activity at the final step was about 41 times in comparison with that of the crude enzyme. FIG. 5 shows the result of SDS-PAGE (CBB staining) on the samples at each step of purification using 10-20% gradient gel, indicating that the purified enzyme preparation (lane 2) is a single protein in SDS-PAGE.

[Table 2]

| | Total protein (mg) | Total activity (U) | Specific activity (u/mg) | Recovery (%) |
|---|---|---|---|---|
| Concentrate | 42 | 300 | 3.9 | 100 |
| Ammonium sulfate fraction | 8.5 | 220 | 22.9 | 73 |
| Phenyl HP | 0.50 | 58 | 100 | 19 |
| Q HP | 0.37 | 53 | 139 | 18 |
| Superdex 200 | 0.12 | 19 | 158 | 6.3 |

2. Properties of maltotriosyl transferase

(1) Optimal reaction temperature

In the same manner as the above-described maltotriosyl transferase activity measurement method, the reaction was carried out at 30°C, 40°C, 45°C, 50°C, 55°C, 60°C, 65°C, 70°C, and 75°C. The results were expressed as relative activity, taking the value at the temperature which gave the maximum activity as 100%. The optimal reaction temperature was in the vicinity of 50°C (FIG. 1).

(2) Optimal reaction pH

In the same manner as the above-described maltotriosyl transferase activity measurement method, the activity was measured under reaction conditions of 40°C and 60 minutes in buffers (universal buffer solution pH 4.0, pH 4.5, pH 5.0, pH 5.5, pH 6.0, pH 6.5, pH 7.0, pH 7.5, pH 8.0, pH 9.0, pH 10.0, and pH 11.0). The results were expressed as relative activity, taking the value at the pH which gave the maximum activity as 100%. The optimal reaction pH was in the vicinity of about 7.5 (FIG. 2).

(3) Thermostability

6 u/mL of enzyme solution was subjected to heat treatment in 10 mmol/L MES buffer solution (pH 6.5) for 30 minutes at 30°C, 40°C, 45°C, 50°C, 55°C, 60°C, 65°C, 70°C, and 75°C, and then the residual activity was measured by the above-described maltotriosyl transferase activity measurement method. The residual activity was expressed taking the activity untreated with heat as 100%. After heat treatment at 65°C for 30 minutes, residual activity was 90% or more, and stable up to 65°C (FIG. 3).

(4) pH stability

6 u/ mL of enzyme solution was treated at 40°C for 30 minutes in each buffer solution (universal buffer solution pH 3.0, pH 4.0, pH 4.5, pH 5.0, pH 5.5, pH 6.0, pH 6.5, pH 7.0, pH 7.5, pH 8.0, pH 9.0, pH 10.0, and pH 11.0), and the activity was measured by the above-described maltotriosyl transferase activity measurement method. In the range from pH 5.0 to pH 10.0, the residual activity was 85% or more, and stable from pH 5.0 to pH 10.0 (FIG. 4).

(5) Molecular weight measurement by SDS-PAGE

SDS-PAGE was carried out in accordance with the method by Laemmli et al. The molecular weight marker used herein was Low Molecular Weight Calibration Kit for Electrophoresis (manufactured by GE Healthcare), which contained Phosphorylase b (97,000 Da), Albumin (66,000 Da), Ovalbumin (45,000 Da), Carbonic anhydrase (30,000 Da), Trypsin inhibitor (20,100 Da), and $\alpha$-Lactalbumin (14,400 Da) as standard proteins. Electrophoresis was carried out for about 80 minutes at 20 mA/gel using a gradient gel (manufactured by Wako Pure Chemical Industries, Ltd.) having a gel concentration of 10-20%, and the molecular weight was determined; the molecular weight was about 83 kDa (FIG. 5).

(6) Isoelectric point

The isoelectric point of the present enzyme was about 4.5 as measured by isoelectric focusing using ampholine (600V, 4°C, a current was passed for 48 hours).

(7) Substrate specificity

The maltotriosyl transferase activity for different substrates was studied.

a) Substrate specificity for maltooligosaccharide

Substrate specificity for maltooligosaccharide was studied as follows. The enzyme was added to each 10 mmol/L maltooligosaccharide to give a concentration of 0.002 u/mL, and allowed to stand at 50°C for 1, 2, and 3 hours. After standing, the mixture was heated for 5 minutes in a boiling water bath, and then cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the consumption rate and production rate were calculated. The rate of reaction for each maltooligosaccharide was calculated as follows. The rate for maltotetraose was the sum of the formation rate of heptaose and triose. The rate for maltopentaose was the sum of the production rate of octaose and triose. The rate for maltohexaose was calculated by halving the difference between the production rates of triose and nonose, and adding the value to the production rate of nonose.

[Table 3]

| Substrate | Relative rate (%) |
|---|---|
| Maltose | 0 |
| Maltotriose | 0 |
| Maltotetrao se | 83 |
| Maltopentaose | 100 |

(continued)

| Substrate | Relative rate (%) |
|---|---|
| Maltohexaose | 89 |

For maltose and maltotriose, no reaction product was found. Sufficient action was observed on maltotetraose, maltopentaose, and maltohexaose.

b) Substrate specificity for polysaccharide

For cyclodextrin, soluble starch, amylose, and amylopectin, the substrate specificity was studied by the following method. The enzyme was added at 0.002 u/mL with reference to 10 mmol/L of each maltooligosaccharide, and 0.1 u/mL of enzyme was allowed to stand at 50°C for 0, 1, 2, and 3 hours. After standing, the mixture was heated for 5 minutes in a boiling water bath, and cooled in running water. To 200 μL of the solution, Rhizopus-derived glucoamylase (manufactured by Wako Pure Chemical Industries, Ltd.) was added at 0.03 mg for 1.0 unit, and allowed to stand at 50°C overnight. After standing, the mixture was heated in a boiling water bath for 5 minutes, and then cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. In the comparison between the groups treated and untreated with the enzyme (maltotriosyl transferase), when the peaks of triose or higher saccharides increased with time, the product was judged as present (+), and when no increase was found, the product was judge as absent (-).

[Table 4]

| Substrate | Presence or absence of product |
|---|---|
| α-cyclodextrin | - |
| β-cyclodextrin | - |
| γ-cyclodextrin | - |
| Amylose | + |
| Amylopectin | + |
| Soluble starch | + |

For cyclodextrin, no reaction product was found. For soluble starch, amylose, and amylopectin, peaks of triose or higher saccharides increased with time. These polysaccharides were found to work as substrates. Since glucoamylase hydrolyzes α-1,4 and α-1,6 bonds, it was found that transglycosylation products were formed for other bonding patterns.

(8) Influence of substrate concentration on enzyme reaction product

The influence of the substrate concentration on the enzyme reaction product was studied using maltotetraose as the substrate. The enzyme was added in such a manner that the residual amount of maltotetraose after reaction for 3 hours was 85% or more with reference to 0.67, 1.0, 3.0, 10, 30, and 70% (w/v) maltotetraose, and allowed to stand at 50°C for 1, 2, and 3 hours. After standing, the mixture was heated in a boiling water bath for 5 minutes, and cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the production rate was calculated. As a result of this, the rate of transglucosylation was 90% or more at all the substrate concentrations (0.67 to 70% (w/v)).

[Table 5]

| Substrate concentration (% (w/v)) | Reaction formation rate (molar ratio) | |
|---|---|---|
| | Transfer product (heptaose) | Decomposition product (triose) |
| 0.67 | 92% | 8% |
| 1.0 | 96% | 4% |
| 3.0 | 100% | 0% |
| 10 | 100% | 0% |
| 30 | 100% | 0% |
| 70 | 100% | 0% |

4. Production of indigestible saccharide

(1) Measurement method of indigestible component content

The measurement was carried out by the improved method of "Determination of Indigestible Components" (Denpun Kagaku (Journal of the Japanese Society of Starch Science), vol. 37, No. 2, p. 107, 1990). Firstly, 1 mL of the sample was adjusted to 15 mL using 50 mmol/L phosphate buffer solution (pH 6.0), 30 μL of α-amylase (equivalent to TERMAMYL 120L manufactured by Wako Pure Chemical Industries, Ltd.) was added, and the mixture was allowed to react at 95°C for 30 minutes. After cooling, the pH was readjusted to 4.5, 30 μL of amyloglucosidase (manufactured by Sigma-Aldrich Co. LLC) was added, and the mixture was allowed to react at 95°C for 30 minutes. The reaction was stopped by boiling in a boiling water bath for 5 minutes. The volume of the reaction solution was adjusted to 30 mL, and the indigestible component content was calculated by the pyranose-oxidase method using the following equation.

$$\text{Indigestible component content } (\%) = 100 - \text{formed amount of glucose } (\%) \times 0.9$$

(2) Preparation of indigestible saccharide by maltotriosyl transferase 1

An indigestible saccharide was produced by adding maltotriosyl transferase to dextrin. Maltotriosyl transferase and a starch hydrolysate (PINEDEX #100 (manufactured by Matsutani Chemical Industry Co,. Ltd., the final concentration 30% (w/v)) were added to 10 mmol/L MES buffer solution (pH 6.0), and the solution amount was adjusted to 20 mL. In order to study the effect of the combination of the maltotriosyl transferase with the other enzyme, the test groups using the combination of the maltotriosyl transferase with cyclodextrin glucanotransferase (TORUZYME 3.0 L manufactured by Novozymes) and/or pullulanase (KLEISTASE PL45 manufactured by Amano Enzyme Inc.) were also prepared.

After reaction at 60°C for 66 hours, the reaction was stopped by boiling in a boiling water bath for 10 minutes. The cooled reaction solution was subjected to the above-described "(1) Measurement method of indigestible component content".

[Table 8]

| Loading of enzyme (/g-substrate) | | | Indigestible component content (%) |
|---|---|---|---|
| G3 transferase | CGTase | Pullulanase | |
| 20 u | 0 | 0 | 49.5 |
| 20 u | 3 mg | 0 | 51.9 |
| 20 u | 10 mg | 0 | 57.9 |
| 20 u | 0.3 mg | 1 mg | 52.1 |
| 20 u | 3 mg | 1 mg | 61.6 |
| 20 u | 10 mg | 1 mg | 63.5 |
| G3 transferase: maltotriosyl transferase<br>CGTase: cyclodextrin glucanotransferase | | | |

Table 8 indicates that the indigestible component contents in the products were 49.5% for the single addition of maltotriosyl transferase (20 u/g (substrate)), 57.9% for the combined addition of maltotriosyl transferase (20 u/g (substrate)) and cyclodextrin glucanotransferase (10 mg/g (substrate)), and 63.5% for the combined addition of three enzymes, or maltotriosyl transferase (20 u/g(substrate)), cyclodextrin glucanotransferase (10 mg/g (substrate)), and pullulanase (1 mg/g (substrate)).

As described above, it was indicated that the maltotriosyl transferase can produce an indigestible saccharide even by the reaction at a high temperature. The reaction at a high temperature is effective for reducing the risk of microorganism contamination, and is particularly demanded for the production on industrial scale. In addition, it was found that the combined use with cyclodextrin glucanotransferase or pullulanase can increase the indigestible saccharide content. In particular, the combined use of three enzymes composed of maltotriosyl transferase, cyclodextrin glucanotransferase, and pullulanase, increased the indigestible saccharide content by 10% or more in comparison with the single use of maltotriosyl transferase.

5. Dietary fiber increasing effect in beer or beer-like beverage

The saccharification reaction used an automatic saccharification tank LB-8 (manufactured by LOCHNER Ltd.). 320 g of 1.2 mmol/L calcium sulfate aqueous solution was charged into the saccharification bath, the temperature was increased to 50°C under stirring, and then 8 g of malt, 72 g of ground barley, and maltotriosyl transferase were added. The loading of the maltotriosyl transferase was 0 u, 96 u or 480 u. After adding the ingredients and the enzyme, the mixture was treated at 50°C for 1 hour. Furthermore, the temperature was increased, kept at 65°C for 1 hour, and then increased again and kept at 76°C for 10 minutes. The saccharified solution thus obtained was filtered through filter paper to obtain a filtrate. The content of the water-soluble dietary fiber in the filtrate was determined by the enzyme-HPLC method. As a result of this, the water-soluble dietary fiber content was 1.7% (w/v) for the enzyme-free group, 2.6% (w/v) for the enzyme 96 u-added group, and 3.2% (w/v) for the 480 u-added group. Dietary fiber will not be utilized by the beer yeast during the subsequent fermentation step. Therefore, the increase of dietary fiber during the saccharification step results in the increase of dietary fiber in the beer. The addition of maltotriosyl transferase increased the dietary fiber in the wort, irrespective of the malt content in the ingredients.

7. Production of hetero-oligosaccharide

Maltotetraose as the donor substrate and a receptor substrate (D-xylose, D-fructose, D-glucose, L-fucose, L-sorbose, D-mannose, D-sorbitol, D-galactose, N-acetylglucosamine, L-arabinose, D-ribose, or L-rhamnose) were dissolved in 10 mmol/L MES buffer solution (pH 6.5) to give a final concentration of 3.0% (w/v) or 10.0% (w/v). 1 u of maltotriosyl transferase was added to 1 mL of the substrate solution, and the mixture was allowed to stand and react in a water bath at 50°C for 24 hours. Subsequently, the reaction was stopped by boiling for 10 minutes. The reaction solution was subjected to HPLC analysis. In the HPLC analysis, the column was CK04S manufactured by Mitsubishi Chemical Corporation, the column temperature was 80°C, the mobile phase was ultrapure water at a flow rate of 0.4 mL/min, and the detector was a differential refractometer. The formation of the product was calculated, taking the formation of the receptor substrate before reaction as 100%. As shown in Table 9, various hetero-oligosaccharides were produced using various receptor substrates in the presence of maltotriosyl transferase. These hetero-oligosaccharides likely have various functions of commercially available functional oligosaccharides, such as anticarious action and intestine regulation action. Examples of the donor substrate other than maltotetraose include dextrin and starch.

[Table 9]

| Receptor substrate | Formation (%) |
|---|---|
| D-xylose | 26.3 |
| D-fructose | 17.0 |
| D-glucose | 15.9 |
| L-fucose | 12.9 |
| L-sorbose | 12.5 |
| D-mannose | 11.3 |
| D-sorbitol | 9.2 |
| D-galactose | 8.2 |

(continued)

| Receptor substrate | Formation (%) |
|---|---|
| N-acetylglucosamine | 8.1 |
| L-arabinose | 7.8 |
| D-ribose | 7.7 |
| L-rhamnose | 3.3 |

8. Production of glycoside 1

Amylose EX-1 as the donor substrate and a receptor substrate (kojic acid, ascorbic acid, or hydroquinone) were dissolved in 5 mmol/L MES buffer solution (pH 6.0) to give a final concentration of 2.5% (w/v). 1 u of maltotriosyl transferase was added to 1 mL of the substrate solution, and allowed to react in a water bath at 50°C for 24 hours. Subsequently, the reaction was stopped by boiling for 10 minutes. The reaction solution was subjected to HPLC analysis. In the HPLC analysis, the column was TSK gel ODS-100V 3 μm (4.6 mm×7.5 cm; manufactured by Tosoh Corporation), the column temperature was 50°C, the flow rate was 1.0 mL/min, and the detector was a UV detector. The mobile phase was composed of a solution A of 0.1% phosphoric acid and a solution B of methanol: ultrapure water = 7:3, and elution was carried out in the pattern of 0 minutes (B: 0%) → 4 minutes (B: 0%) → 8 minutes (B: 100%) → 10 minutes (B: 100% → B: 0%) → 15 minutes (B: 0%). The formation of the product was calculated, taking the molar amount of the receptor substrate before reaction as 100%. Various glycosides were produced by the use of maltotriosyl transferase. Sugar bonding by maltotriosyl transferase allows the production of a glycoside having improved functions and properties, such as improved solubility and stability of the substance.

[Table 10]

| Receptor substrate | Formation (%) |
|---|---|
| Kojic acid | 6.1 |
| Ascorbic acid | 1.9 |
| Hydroquinone | 1.5 |

9. Production of glycoside 2

Maltotetraose as the donor substrate and glycerol as the receptor substrate were dissolved in 10 mmol/L MES buffer solution (pH 6.5) to give a final concentration of 3.0% (w/v) or 10.0% (w/v). 1 u of maltotriosyl transferase was added to 1 mL of the substrate solution, and allowed to stand and react in a water bath at 50°C for 24 hours. Subsequently, the reaction was stopped by boiling for 10 minutes. The reaction solution was subjected to HPLC analysis. In the HPLC analysis, the column was CK04S (manufactured by Mitsubishi Chemical Corporation), the column temperature was 80°C, the mobile phase was ultrapure water at a flow rate of 0.4 mL/min, and the detector was a differential refractometer. The formation of the product was calculated, taking the amount of the receptor substrate before reaction as 100%. Through the use of maltotriosyl transferase, a glycerol glycoside was produced in a molar amount of 7.3% with reference to the receptor substrate. The glycerol glycoside is a non-reducing glycoside contained in sake mash, mirin (Japanese sweet rice wine for cooking), and miso, and is said to impart sharp sweetness and richness to Japanese sake. In addition, the degree of sweetness of the glycoside is half that of sugar, and the glycoside can be added to food and beverages as a sweetener which has a low browning potential, low Maillard reactivity, high thermal stability, anticarious effect, indigestibility, low hygroscopicity, and high moisture retentivity.

[Industrial Applicability]

The present application discloses a method for producing an indigestible saccharide. The indigestible saccharide obtained by the production method of the present invention is expected to find uses in the food and drug fields.

## Claims

1. A method for producing an indigestible saccharide, comprising a step of allowing maltotriosyl transferase to act on a saccharide, wherein the saccharide is dextrin.

2. The production method according to claim 1, wherein the step is carried out using one or more enzymes selected from the group consisting of cyclodextrin glucanotransferase, α-amylase (only those having transglycosylation activity), pullulanase, and isoamylase in combination with the maltotriosyl transferase.

3. The production method according to claim 1 or 2, wherein the reaction temperature during the step is from 50°C to 70°C.

4. The method for producing an indigestible saccharide according to any one of claims 1 to 3, wherein the step is carried out in the presence of a receptor substrate, and a hetero-oligosaccharide is formed as the indigestible saccharide.

5. The method for producing an indigestible saccharide according to claim 4, wherein the receptor substrate is a monosaccharide and/or a sugar alcohol.

6. The production method according to claim 4, wherein the receptor substrate is one or more sugars or sugar alcohols selected from the group consisting of D-xylose, D-fructose, D-glucose, L-fucose, L-sorbose, D-mannose, D-sorbitol, D-galactose, N-acetyl glucosamine, L-arabinose, D-ribose, and L-rhamnose.

7. The method for producing an indigestible saccharide according to claim 1, wherein the saccharide is formed by saccharification of a saccharide ingredient used for producing beer or a beer-like beverage, preferably wherein the saccharide ingredient is one or more ingredients selected from the group consisting of malt, barley, wheat, rye, oat, corn, rice, kaoliang, potato, soybean, pea bean, chick-pea, and corn starch.

## Patentansprüche

1. Verfahren zur Herstellung eines unverdaulichen Saccharids umfassend einen Schritt des Ermöglichens, dass Maltotriosyl-Transferase auf ein Saccharid wirkt, wobei das Saccharid Dextrin ist.

2. Herstellungsverfahren nach Anspruch 1, wobei der Schritt unter Verwendung einer oder mehrerer Enzyme auswählt aus der Gruppe bestehend aus Cyclodextrin-Glucanotransferase, α-Amylase (nur die, die Transglycosylierungsaktivität aufweisen), Pullulanase und Isoamylase in Kombination mit der Maltotrosyl-Transferase durchgeführt wird.

3. Herstellungsverfahren nach Anspruch 1 oder 2, wobei die Reaktionstemperatur während des Schritts zwischen 50°C und 70°C liegt.

4. Verfahren zur Herstellung eins unverdaulichen Saccharids nach einem der Ansprüche 1 bis 3, wobei der Schritt in Anwesenheit eines Rezeptorsubstrats durchgeführt wird und ein Heterooligosaccharid als das unverdauliche Saccharid gebildet wird.

5. Verfahren zur Herstellung eines unverdaulichen Saccharids nach Anspruch 4, wobei das Rezeptorsubstrat ein Monosaccharid und/oder ein Zuckeralkohol ist.

6. Herstellungsverfahren nach Anspruch 4, wobei das Rezeptorsubstrat ein oder mehrere Zucker oder Zuckeralkohole ausgewählt aus der Gruppe bestehend aus D-Xylose, D-Fruktose, D-Glukose, L-Fukose, L-Sorbose, D-Mannose, D-Sorbitol, D-Galactose, N-Acetyl-Glucosamin, L-Arabinose, D-Ribose und L-Rhamnose ist.

7. Verfahren zur Herstellung eines unverdaulichen Saccharids nach Anspruch 1, wobei das Saccharid durch Verzuckerung eines Saccharidbestandteils gebildet wird, das zur Herstellung von Bier oder bierähnlichen Getränken verwendet wird, vorzugsweise wobei der Saccharidbestandteil ein oder mehrere Bestandteile ausgewählt aus der Gruppe bestehend aus Malz, Gerste, Weizen, Roggen, Hafer, Mais, Reis, Kaoliang, Kartoffel, Sojabohne, Pea bean , Kichererbse und Maisstärke ist.

## Revendications

1. Procédé pour produire un saccharide indigeste, comprenant une étape de faire agir une maltotriosyl transférase sur un saccharide, où le saccharide est une dextrine.

2. Procédé de production selon la revendication 1, où l'étape est accomplie au moyen d'une ou plusieurs enzymes choisies dans le groupe consistant en cyclodextrine glucanotransférase, α-amylase (seulement celles ayant une activité de transglycosylation), pullulanase et isoamylase en combinaison avec la maltotriosyl transférase.

3. Procédé de production selon la revendication 1 ou 2, où la température de réaction pendant l'étape est de 50°C à 70°C.

4. Procédé pour produire un saccharide indigeste selon l'une quelconque des revendications 1 à 3, où l'étape est accomplie en présence d'un substrat récepteur, et un hétéro-oligosaccharide est formé comme saccharide indigeste.

5. Procédé pour produire un saccharide indigeste selon la revendication 4, où le substrat récepteur est un monosaccharide et/ou un sucre-alcool.

6. Procédé de production selon la revendication 4, où le substrat récepteur est un ou plusieurs sucres ou sucres-alcools choisis dans le groupe consistant en le D-xylose, le D-fructose, le D-glucose, le L-fucose, le L-sorbose, le D-mannose, le D-sorbitol, le D-galactose, la N-acétylglucosamine, le L-arabinose, le D-ribose et le L-rahmnose.

7. Procédé pour produire un saccharide indigeste selon la revendication 1, où le saccharide est formé par saccharification d'un ingrédient saccharidique utilisé pour produire la bière ou une boisson analogue à la bière, de préférence où l'ingrédient saccharidique est un ou plusieurs ingrédients choisis dans le groupe consistant en le malt, l'orge, le blé, le seigle, l'avoine, le maïs, le riz, le kaoliang, la pomme de terre, le soja, le petit haricot rond, le pois chiche et l'amidon de maïs.

Fig. 1

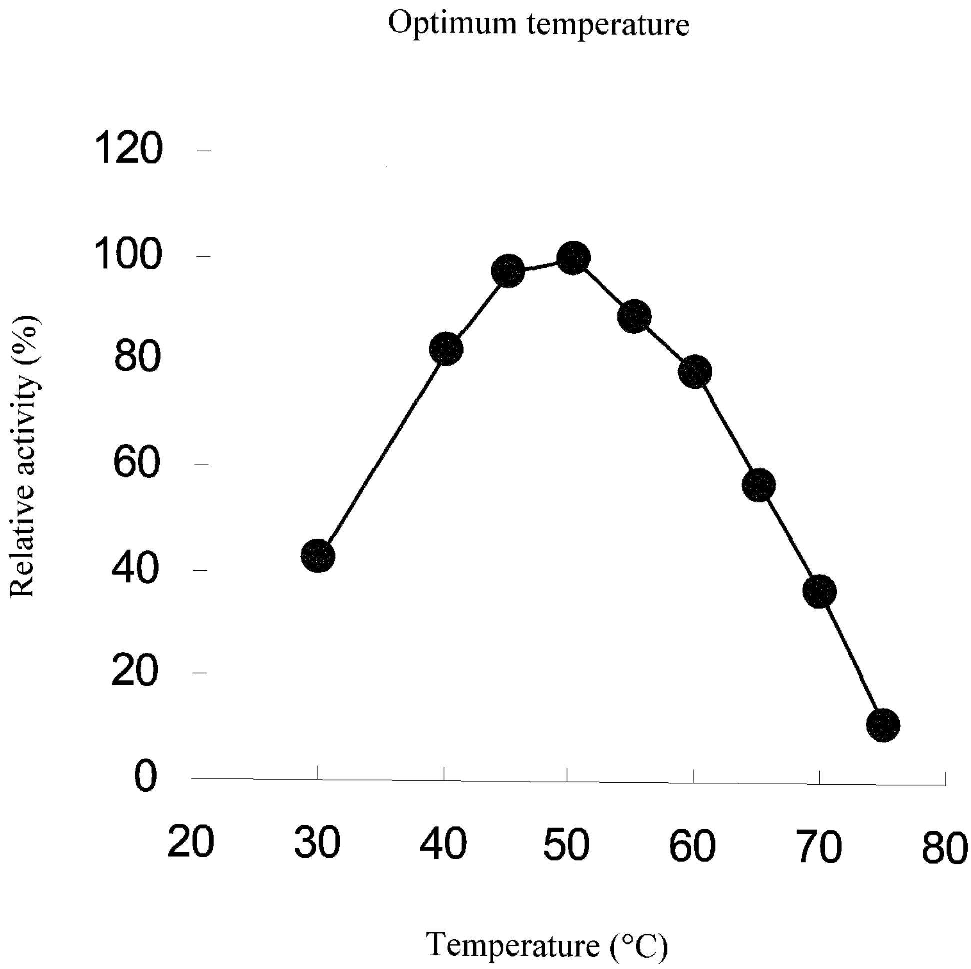
Optimum temperature
120
100
80
60
40
20
0
20
30
40
50
60
70
80
Relative activity (%)
Temperature (°C)

Fig. 2

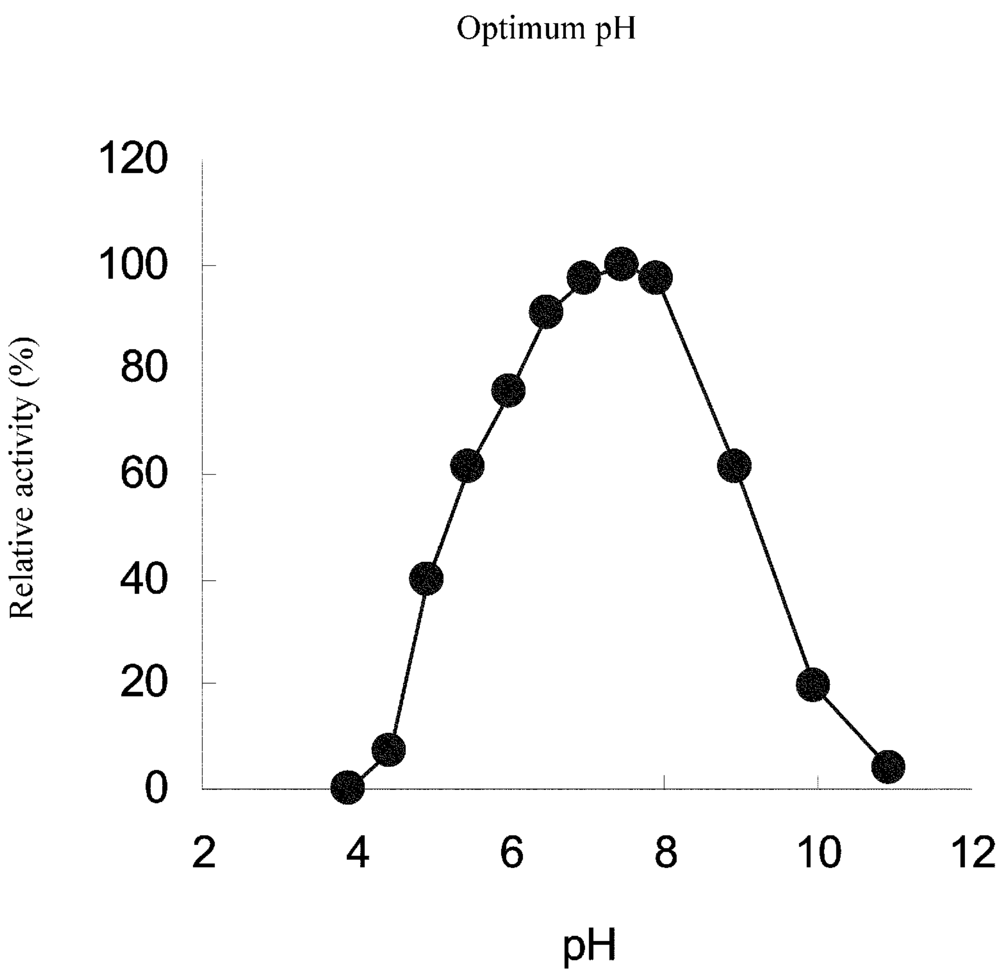
Optimum pH
120
100
80
60
40
20
0
Relative activity (%)
2
4
6
8
10
12
pH

Fig. 3

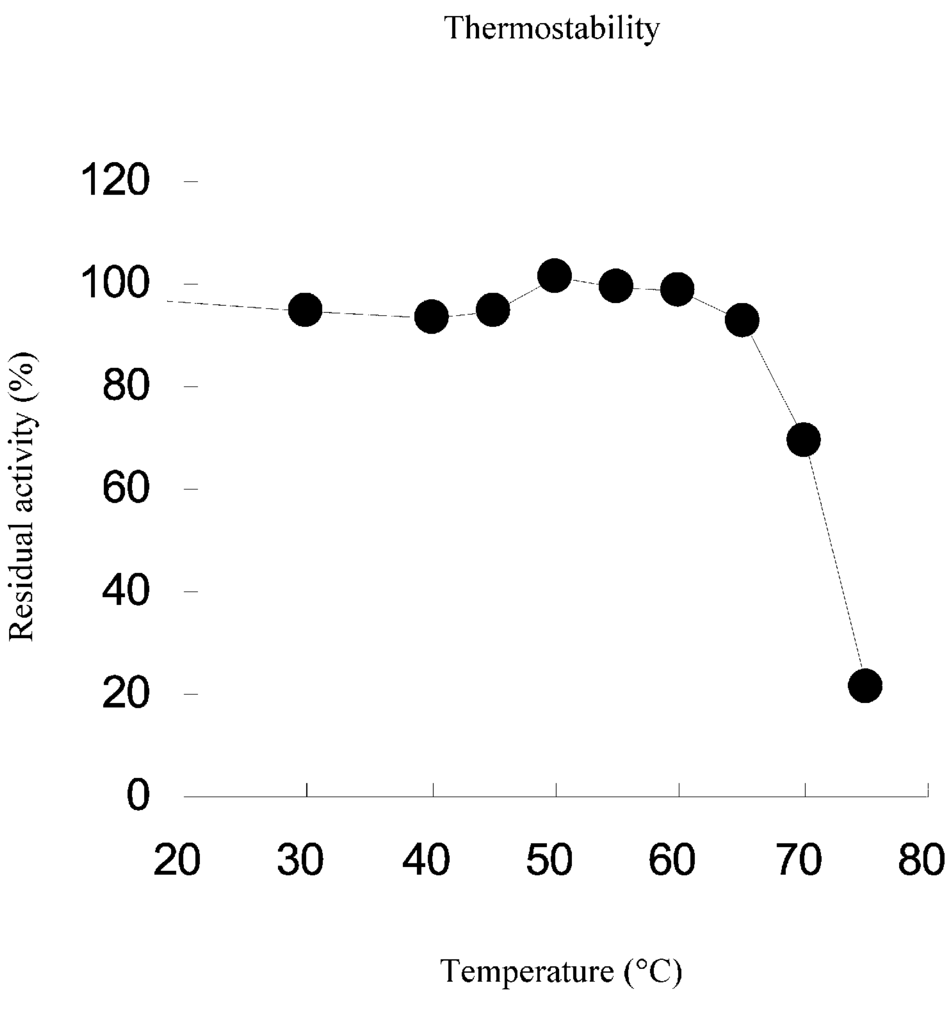
Thermostability
120
100
80
60
40
20
0
Residual activity (%)
20
30
40
50
60
70
80
Temperature (°C)

Fig. 4

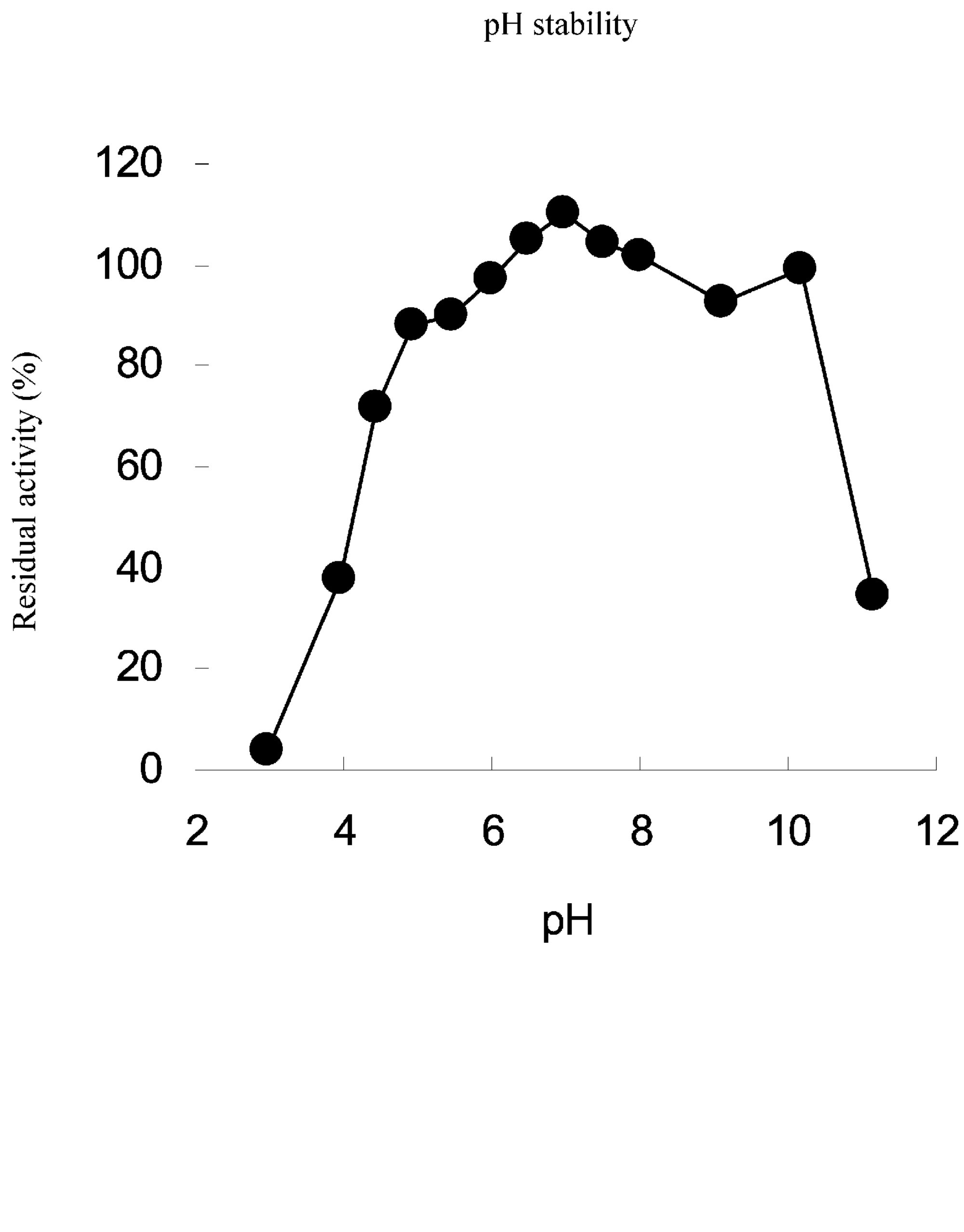
pH stability
Residual activity (%)
120
100
80
60
40
20
0
2
4
6
8
10
12
pH

Fig. 5

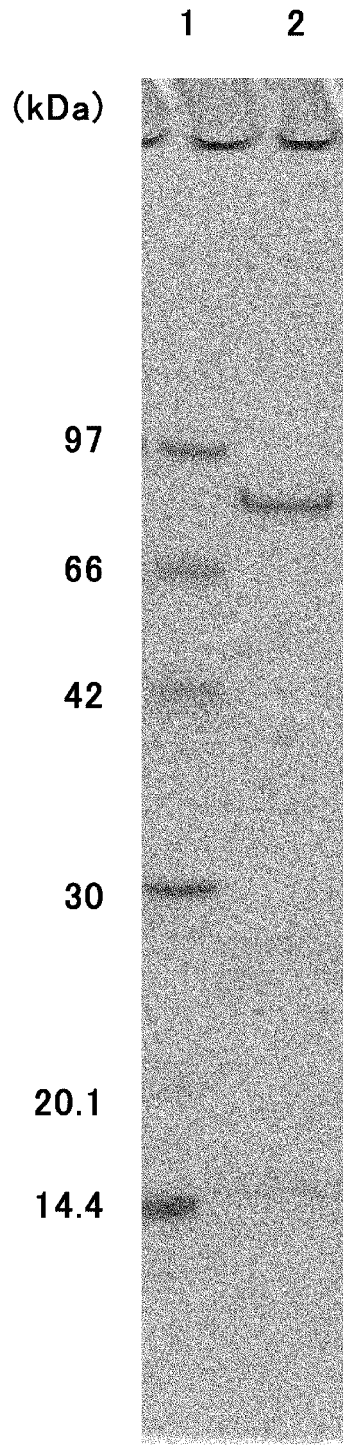

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001294601 A
- WO 2011001722 A
- JP 54049354 A
- JP 2008194024 A
- JP 9107900 A
- WO 2008136331 A
- US 2005031734 A1
- JP P2011023281 B
- JP P2011162973 B


### Non-patent literature cited in the description


- **OKADA et al.** *Denpun Kagaku (Journal of the Japanese Society of Starch Science),* 1983, vol. 30 (2), 223-230
- **KARLIN ; ALTSCHUL.** Proc. Natl. Acad. Sci. USA. 1990, vol. 87, 2264-68
- **KARLIN ; ALTSCHUL.** *Proc. Natl. Acad. Sci. USA,* 1993, vol. 90, 5873-77
- **ALTSCHUL et al.** *J. Mol. Biol.,* 1990, vol. 215, 403-10
- **ALTSCHUL et al.** *Amino Acids Research,* 1997, vol. 25 (17), 3389-3402
- **MYERSAND MILLER.** *Comput Appl BioSci.,* 1988, vol. 4, 11-17
- Determination of Indigestible Components. Journal of the Japanese Society of Starch Science. Denpun Kagaku, 1990, vol. 37, 107